# EUROPÄISCHE PATENTSCHRIFT

(11) **EP 1 056 409 B1**
(45) Veröffentlichungstag und Bekanntmachung des Hinweises auf die Patenterteilung: **06.10.2004**
(21) Anmeldenummer: 99973468.4
(22) Anmeldetag: 06.11.1999
(51) Int. Cl.: A61B 18/14

(54) **BIPOLARES MEDIZINISCHES INSTRUMENT**
BIPOLAR MEDICAL INSTRUMENT
INSTRUMENT BIPOLAIRE A USAGE MEDICAL

(30) Priorität: 18.12.1998 DE 19858512
(43) Veröffentlichungstag der Anmeldung: 06.12.2000
(73) Patentinhaber: Karl Storz GmbH & Co. KG, 78532 Tuttlingen (DE)
(72) Erfinder: DORN, Jürgen, D-68809 Neulussheim (DE)
(74) Vertreter: Heuckeroth, Volker, Dr.
(86) Internationale Anmeldenummer: PCT/EP1999/008517
(87) Internationale Veröffentlichungsnummer: WO 2000/036986

(56) Entgegenhaltungen:
- WO-A-95/05125
- DE-C- 4 421 822
- FR-A- 2 647 683

## Beschreibung

Die Erfindung betrifft ein bipolares medizinisches Instrument, mit einem Rohrschaft, mit zwei Maulteilen am distalen Ende des Rohrschafts, wobei die Maulteile gelenkig miteinander verbunden sind und jeweils eine mit Hochfrequenzstrom beaufschlagbare Elektrode ausbilden.

Ein derartiges bipolares medizinisches Instrument ist aus der DE 196 08 716 C1 bekannt.

Ein solches Instrument wird im Rahmen der minimal-invasiven Chirurgie zur Durchführung endoskopischer Operationen im menschlichen oder tierischen Körper verwendet.

Die beiden Maulteile am distalen Ende des Rohrschafts sind gelenkig miteinander verbunden, so daß die beiden Maulteile durch Betätigen einer Handhabe am proximalen Ende des Rohrschafts geschlossen und geöffnet werden können. Mit den Maulteilen wird Gewebe im Körper geschnitten und/oder gefaßt, um Gewebe im Körper abzutrennen und zu entfernen oder beiseite zu legen. Entsprechend sind die Maulteile als Schneidwerkzeuge mit Schneidkanten oder als Faßwerkzeuge mit beim Schließen der Maulteile stumpf aufeinander stoßenden Flächen ausgebildet.

Von den beiden Maulteilen ist zumindest eines gelenkig mit dem Rohrschaft verbunden, während das andere Maulteil mit dem Rohrschaft starr oder ebenfalls gelenkig verbunden ist.

Weiterhin ist es bei der eingangs genannten Art eines medizinischen Instruments vorgesehen, daß beide Maulteile jeweils eine mit Hochfrequenzstrom beaufschlagbare Elektrode ausbilden. Beide Maulteile können getrennt voneinander mit jeweils einem Pol einer Hochfrequenzspannungsquelle verbunden werden. Durch Beaufschlagen der beiden Maulteile mit bipolarem Hochfrequenzstrom kann einerseits im Falle eines Schneidwerkzeuges die Schneidwirkung durch die thermische Wirkung des Hochfrequenzstroms in dem Gewebe erhöht werden, andererseits kann im Falle eines Faßwerkzeuges durch die Wärmeentwicklung eine Koagulation des zwischen den Maulteilen gefaßten Gewebes erreicht werden.

Da bei derartigen Instrumenten die Maulteile, das Gelenk, über die die Maulteile miteinander verbunden sind, und der Rohrschaft in der Regel aus Metall und damit elektrisch leitend ausgeführt sind, besteht ein Problem darin, eine elektrische Isolierung zwischen beiden, jeweils eine Elektrode ausbildenden Maulteilen zu bewerkstelligen, um beim Beaufschlagen der Maulteile mit Hochfrequenzstrom einen Kurzschluß zu vermeiden, da beide Maulteile an unterschiedliche Potentiale gelegt werden. Das Problem der elektrischen Trennung der beiden Maulteile voneinander stellt sich als um so größer dar, je kleiner ein derartiges Instrument im Bereich der Maulteile und damit im Bereich des Gelenkes ausgebildet wird, insbesondere, wenn der Durchmesser des Instruments im Bereich des Gelenks 5 mm und weniger beträgt.

Bei dem aus der eingangs genannten DE 196 08 716 C1 bekannten Instrument wird die elektrische Isolierung der beiden Maulteile voneinander dadurch bewerkstelligt, daß in die ansonsten metallisch ausgeführte gelenkige Verbindung der beiden Maulteile Keramikelemente eingesetzt werden, die also ein Teil des Gelenkes selbst bilden. Diese Art der elektrischen Isolierung der beiden Maulteile voneinander im Bereich des Gelenks hat jedoch den Nachteil, daß bei einer Miniaturisierung dieses Instruments auch die Keramikelemente in ihrer Stärke reduziert werden müssen. Da üblicherweise an die Maulteile eine Hochfrequenzspannung in der Größenordnung von 2,5 kV gelegt wird, bedeutet dies, daß bei einer Reduzierung der Stärke der Keramikelemente ein Durchschlagen der Spannung durch das Keramikelement hindurch auftreten kann. Ein weiterer Nachteil der elektrischen Isolierung der beiden Maulteile voneinander im Bereich des Gelenks besteht darin, daß die eingesetzten Keramikelemente beim Bewegen der Maulteile mitbewegt werden, und somit aufgrund von Reibung im Laufe der Zeit zerrieben werden können.

Weiterhin ist aus der DE 43 12 284 A1 ein bipolares medizinisches Instrument bekannt, bei dem die Maulteile insgesamt aus Kunststoff bestehen, in den Endabschnitte von Stromzuleitungen eingebettet sind. Hierbei ist von Nachteil, daß die Maulteile bei Beaufschlagen mit Hochfrequenzstrom der dabei entstehenden hohen Wärmeentwicklung mitunter nicht standhalten können.

Aus dem Dokument FR-A-2 647 683 ist eine bipolare medizinische Pinzette bekannt, die zwei Pinzettenarme aufweist, die an ihrem proximalen Ende seitlich voneinander beabstandet in einem Halter befestigt sind. Die Pinzettenarme sind aufgrund ihrer länglichen Ausgestaltung elastisch, so daß ihre distalen Enden durch Zusammendrücken der Pinzettenarme in ihrem isolierten proximalen Bereich geschlossen werden können. Die distalen Enden der Pinzettenarme sind metallisch ausgebildet und weisen auf ihren einander zugewandten Seiten jeweils ein Isolatorelement auf, wobei in den beiden Isolatorelementen jeweils eine Vielzahl von Öffnungen enthalten ist, in denen ein elektrisch leitendes Harz eingegossen und ausgehärtet ist. Durch die Öffnungen in dem jeweiligen Isolatorelement hindurch steht das Harz mit dem metallischen Grundkörper in elektrischer Verbindung, wobei der metallische Grundkörper als Stromzuführung für die Vielzahl an Harzelektroden dient. In einem weiteren Ausführungsbeispiel in diesem Dokument ist auf den einander abgewandten Seiten der Metallkörper eine isolierende Beschichtung vorhanden, durch die ein Stromleiter hindurchführt, die eine am distalen Ende an dem Isolatorelement zusätzlich vorgesehene Elektrode mit Strom versorgt.

Der Erfindung liegt daher die Aufgabe zugrunde, ein bipolares medizinisches Instrument der eingangs genannten Art dahingehend weiterzubilden, daß insbesondere bei einer miniaturisierten Ausgestaltung des Instruments im Bereich der Maulteile und der gelenkigen Verbindung der beiden Maulteile eine sichere elektrische Isolation mit konstruktiv einfachen Mitteln erreicht wird.

Hinsichtlich der eingangs genannten bipolaren medizinischen Instrumente wird diese Aufgabe erfindungsgemäß durch das Gerät gemäß Anspruch 1 gelöst.

Anstatt wie bei dem aus dem Stand der Technik bekannten Instrument die elektrische Isolation der beiden Maulteile im Gelenk zu realisieren, ist bei dem erfindungsgemäßen Instrument vorgesehen, die elektrische Isolation an den Maulteilen selbst vorzunehmen. Dabei sind jedoch die Maulteile nicht, wie ebenfalls im Stand der Technik vorgesehen, insgesamt aus einem isolierenden Material, beispielsweise aus Kunststoff, ausgebildet, sondern weisen jeweils einen metallischen Grundkörper auf, wodurch die Maulteile vorteilhafterweise die für solche Instrumente erforderliche hohe mechanische Festigkeit besitzen. Die elektrische Isolation wird durch die Isolatorelemente bewerkstelligt, die auf den einander zugewandten Seiten der Grundkörper angeordnet sind, und somit eine elektrische Trennung der beiden Grundkörper voneinander bewirken. Um die mit Hochfrequenzstrom beaufschlagbaren Elektroden auszubilden, ist jeweils ein Leiterelement mit dem zugehörigen Isolatorelement verbunden, wobei die Strombeaufschlagung durch die durch den Rohrschaft verlaufenden, voneinander isolierten Stromzuleitungen bewirkt wird, die bis zu den Leiterelementen reichen und mit diesen leitend verbunden sind. Die Leiterelemente sind von den metallischen Grundkörpern durch die Isolatorelemente getrennt, so daß die Maulteile, die über die Grundkörper gelenkig miteinander verbunden sind, gegeneinander isoliert sind.

Im Unterschied zum Stand der Technik kann die gelenkige Verbindung der beiden Maulteile durchweg metallisch und damit elektrisch leitend ausgebildet werden, wodurch Raum ergreifende Isolierungsmaßnahmen im Bereich des Gelenks vermieden werden und das erfindungsgemäße Instrument somit ohne Stabilitätsverlust des Gelenkes besonders schmal bauend ausgeführt werden kann, weil Isolationsmaterialien, wie Keramiken, die nicht die gleiche mechanische Beständigkeit aufweisen wie Metalle, als Bauelemente des Gelenkes vermieden werden.

Somit wird die der Erfindung zugrunde liegende Aufgabe vollkommen gelöst.

In einer bevorzugten Ausgestaltung ist ein distales Ende der jeweiligen Stromzuleitung im zugehörigen Isolatorelement von diesem umschlossen angeordnet und mit einem in das Isolatorelement ragenden Fortsatz des Leiterelements leitend verbunden.

Hierbei ist von Vorteil, daß die jeweilige Stromzuleitung im Isolatorelement einerseits mechanisch sicher verankert ist, und daß andererseits auch das Leiterelement durch den in das Isolatorelement ragenden Fortsatz mechanisch sicher mit dem Isolatorelement verbunden ist.

Dabei ist es weiterhin bevorzugt, wenn das distale Ende der jeweiligen Stromzuleitung im Isolatorelement von dessen proximalem bis zu dessen distalem Ende durchgeführt und in ein in das distale Ende des Isolatorelements ragende Röhrchen des Leitelements eingeschoben ist.

Durch die durchgängige Einbettung der Stromzuleitung in das Isolatorelement wird der Vorteil erzielt, daß die Stromzuleitung noch besser in dem Isolatorelement verankert werden kann. Der weitere Vorteil besteht darin, daß die Stromzuleitung, die in Form eines dünnen mit einem isolierenden Mantel umgebenden Drahts ausgebildet sein kann, in das proximale Ende des Isolatorelements zusammen mit ihrem isolierenden Mantel eingeführt werden kann, so daß eine Berührung der Stromzuleitung mit dem metallischen Grundkörper des jeweiligen Maulteils sicher vermieden wird. Das äußerste distale Ende der Stromzuleitung, das dann entisoliert ist, wird in das Isolatorelement ragende Röhrchen des Leitelements eingeschoben, wodurch eine sichere elektrisch leitende Verbindung der Stromzuleitung mit dem Leitelement bewerkstelligt wird.

In einer weiteren bevorzugten Ausgestaltung überragen die Isolatorelemente den zugehörigen metallischen Grundkörper distal, und sind die Leiterelemente am distalen Ende der Isolatorelemente angeordnet und weisen eine Spitze auf.

Bei dieser Ausgestaltung befindet sich die wirksame Elektrodenfläche der beiden Maulteile an deren distalen Ende, so daß mit der Spitze Gewebe unter der Wirkung von Hochfrequenzstrom präpariert werden kann. Dadurch, daß das Isolatorelement den zugehörigen metallischen Grundkörper distal überragt, wird eine sichere Trennung der Leiterelemente, d.h. der Elektroden, und der metallischen Grundkörper erreicht.

In einer weiteren bevorzugten Ausgestaltung enden die Spitzen distal in pinzettenähnlichen Branchen.

Diese Maßnahme hat den Vorteil, daß mit den pinzettenähnlichen Branchen ein besonders feines Präparieren unter Hochfrequenzstromeinwirkung ermöglicht wird. Durch die spitzen Branchen entsteht aufgrund eines Spitzeneffektes eine hohe Hochfrequenzstrom-Leistungsdichte, so daß damit auch eine Schneidfunktion bzw. ein schneidenähnliches Verhalten des Instruments ermöglicht wird, ohne daß Scherenschneiden vorgesehen sein müssen.

Dabei ist es bevorzugt, wenn die Leiterelemente das distale Ende der Isolatorelemente als Kappe umgeben.

Hierbei ist von Vorteil, daß die Leiterelemente das distale Ende der Isolatorelemente schützend umgeben, so daß das distale Ende des Isolatorelements, das beispielsweise aus einer Keramik besteht und daher weniger abriebfest ist, gegen Abnutzung geschützt wird.

In einer weiteren bevorzugten Ausgestaltung sind die Leiterelemente als Plättchen ausgebildet und erstrecken sich flächig im wesentlichen über die gesamte, dem jeweils gegenüberliegenden Maulteil zugewandte Oberfläche der Isolatorelemente.

Durch diese Maßnahme wird die wirksame Elektrodenfläche der beiden Maulteile vorteilhaft vergrößert, so daß zwischen den Maulteilen gefaßtes Gewebe großflächig mit Hochfrequenzstrom behandelt, d.h. koaguliert werden kann.

In einer weiteren bevorzugten Ausgestaltung überragen die Isolatorelemente den metallischen Grundkörper in Umfangsrichtung.

Durch diese Maßnahme wird auf konstruktiv vorteilhaft einfache Weise eine genügend große Beabstandung der beiden metallischen Grundkörper auch an deren Längsseiten im Bereich der als Elektroden wirkenden Leiterelemente erreicht, so daß auch bei hohen Spannungen ein Funkenschlag vermieden wird.

In einer weiteren bevorzugten Ausgestaltung sind die Isolatorelemente von dem jeweiligen metallischen Grundkörper eingefaßt.

Hierbei ist von Vorteil, daß die Isolatorelemente auch bei hohen mechanischen Beanspruchungen, wie sie beispielsweise beim Schneiden oder Fassen von Gewebe beim Schließen der Maulteile auftreten können, ablösungssicher von dem metallischen Grundkörper gehalten werden.

In einer weiteren bevorzugten Ausgestaltung sind die Isolatorelemente mit dem metallischen Grundkörper verklebt.

Hierdurch ergibt sich eine besonders einfache und stabile ablösungssichere Verbindung des Isolatorelements mit dem metallischen Grundkörper.

Dabei ist es bevorzugt, wenn der Klebstoff hitze- und/oder feuchtigkeitsbeständig ist.

Hierbei ist von Vorteil, daß sich die Verbindung zwischen dem Isolatorelement und dem metallischen Grundkörper auch bei der im Gebrauch des Instruments auftretenden Wärmeentwicklung nicht löst, wobei der weitere Vorteil darin besteht, daß die Maulteile in einem Autoklaven bei hohen Dampfdrücken und hohen Temperaturen sterilisiert werden kann, so daß das erfindungsgemäße Instrument den hohen Anforderungen hinsichtlich der Reinigbarkeit genügt.

In einer weiteren bevorzugten Ausgestaltung sind die Isolatorelemente aus einem Keramikwerkstoff, vorzugsweise einem Keramikwerkstoff hoher Härte und geringer Sprödigkeit gefertigt.

Hierbei ist von Vorteil, daß das Isolatorelement jedes Maulteils eine hohe mechanische Beständigkeit besitzt. Da die Isolatorelemente bei dem erfindungsgemäßen Instrument im Wirkbereich der Maulteile angeordnet sind und somit Teil des Faß- oder Schneidwerkzeugs selbst sind, wird dadurch eine auch hohen Kraftbeanspruchungen genügende Beständigkeit der Maulteile erzielt.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird hiernach mit Bezug auf diese näher beschrieben. Es zeigen:
- Fig. 1: eine Gesamtseitenansicht eines bipolaren medizinischen Instruments;
- Fig. 2: einen Längsschnitt durch das distale Ende des Instruments in Fig. 1 in einem vergrößerten Maßstab; und
- Fig. 3: einen Querschnitt entlang der Linie III-III in Fig. 2 durch eines der Maulteile des Instruments.

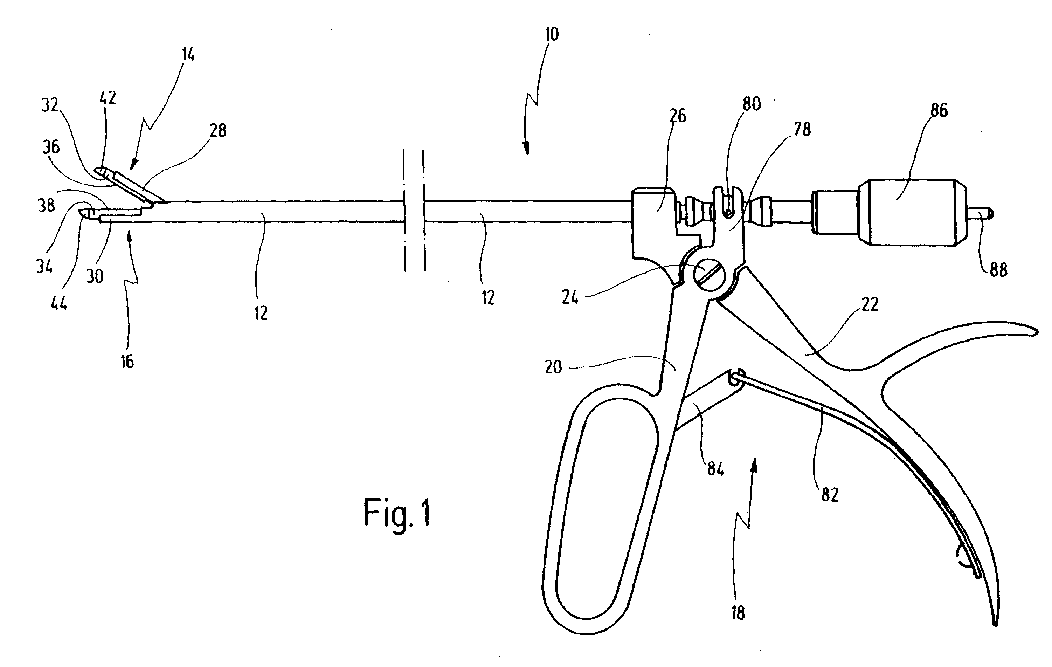

In Fig. 1 bis 3 ist ein mit dem allgemeinen Bezugszeichen 10 versehenes bipolares medizinisches Instrument dargestellt. Das Instrument 10 dient in der minimalinvasiven Chirurgie zum Präparieren von Gewebe im menschlichen oder tierischen Körper, wobei das Instrument 10 gemäß dem Ausführungsbeispiel als Faßinstrument zum Fassen von Gewebe im menschlichen oder tierischen Körper unter endoskopischer Kontrolle verwendet wird. Das Instrument 10 kann speziell zum Koagulieren des gefaßten Gewebes unter der Wirkung von Hochfrequenzstrom eingesetzt werden.

Das Instrument 10 weist einen langerstreckten Rohrschaft 12 auf, an dessen distalen Ende ein erstes Maulteil 14 und ein zweites Maulteil 16 angeordnet sind.

Die Maulteile 14 und 16 sind, wie hiernach noch näher beschrieben wird, gelenkig miteinander verbunden. Dabei ist das erste Maulteil 14 beweglich, während das zweite Maulteil 16 starr mit dem Rohrschaft 12 verbunden ist. Im Rahmen der Erfindung ist jedoch auch eine Ausgestaltung möglich, bei der sowohl das Maulteil 14 als auch das Maulteil 16 beweglich sind.

Am proximalen Ende des Rohrschafts 12 weist das Instrument 10 eine Handhabe 18 auf, die ein erstes Griffelement 20 und ein zweites Griffelement 22 aufweist. Das erste Griffelement 20 ist mit dem zweiten Griffelement 22 über ein Gelenk 24 verbunden, so daß die Griffelemente 20 und 22 relativ zueinander beweglich sind. Dabei ist das erste Griffelement 20 relativ zum Rohrschaft 12 beweglich, während das zweite Griffelement 22 einen Schenkelabschnitt 26 aufweist, der mit dem Rohrschaft 12 fest verbunden ist.

Das erste Maulteil 14 sowie das zweite Maulteil 16 weisen jeweils einen metallischen Grundkörper 28 bzw. 30 auf. Der metallische Grundkörper 30 des zweiten Maulteils 16 ist dabei einstückig mit dem Rohrschaft 12 verbunden, während der metallische Grundkörper 28 relativ zu diesem beweglich ist.

Auf ihrer einander zugewandten Seite weisen die metallischen Grundkörper 28 bzw. 30 jeweils ein Isolatorelement 32 bzw. 34 auf. Die Isolatorelemente 32 und 34 sind aus einem Keramikwerkstoff gefertigt, der eine hohe Härte und geringe Sprödigkeit aufweist.

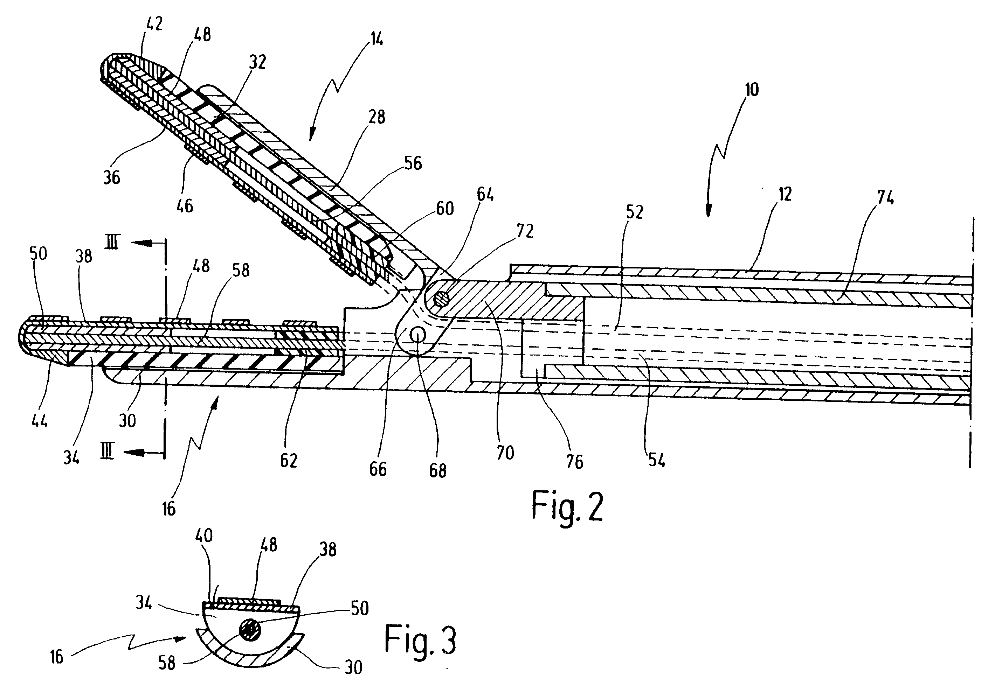

Wie aus Fig. 3 hervorgeht, die einen Schnitt durch das Maulteil 16 zeigt, ist das Isolatorelement 34 in den metallischen Grundkörper 30 eingefaßt, indem der metallische Grundkörper 30 eine teilkreisförmige konkave Aufnahme für das daran formschlüssig angepaßte Isolatorelement 34 bildet. Die gleiche Ausgestaltung weist der metallische Grundkörper 28 auf, mit dem das Isolatorelement 32 des ersten Maulteils 14 verbunden ist.

Die Isolatorelemente 32 und 34 sind in ihren zugehörigen metallischen Grundkörper 28 bzw. 30 eingeklebt. Dazu wird ein Klebstoff verwendet, der hitze- und/oder feuchtigkeitsbeständig ist.

Mit den Isolatorelementen 32 bzw. 34 ist jeweils ein, eine Elektrode bildendes Leiterelement 36 bzw. 38 verbunden. Die Leiterelemente 36 und 38 sind metallisch und somit elektrisch leitend ausgebildet und so an den Isolatorelementen 32 und 34 angeordnet, daß sie den jeweiligen metallischen Grundkörper 28 bzw. 30 nicht berühren, sondern von diesem allseitig beabstandet sind.

Die Leiterelemente 36 und 38 weisen die Form von Plättchen auf, die sich über die gesamte Breite (vergleiche Fig. 3) und gesamte Länge (vergleiche Fig. 2) der einander gegenüber liegenden Oberflächen der Isolatorelemente 32 und 34 erstrecken. Wie aus Fig. 3 hervorgeht, ist diese Oberfläche der Isolatorelemente 32 bzw. 34, die in Fig. 3 bei dem Isolatorelement 34 mit dem Bezugszeichen 40 versehen ist, eben.

Wie aus Fig. 2 ferner hervorgeht, überragt das Isolatorelement 32 bzw. das Isolatorelement 34 den zugehörigen metallischen Grundkörper 28 bzw. 30 distal, wobei das jeweilige Leiterelement 36 bzw. 38 auch am jeweiligen distalen Ende des Isolatorelements 32 bzw. 34 angeordnet ist und dort eine Spitze 42 bzw. 44 aufweist. Die metallischen und somit elektrisch leitenden Spitzen 42 bzw. 44 sind mit dem übrigen plättchenförmigen Abschnitt der Leiterelemente 36 bzw. 38 einstückig verbunden. Die Spitzen 42 und 44 umgeben das distale Ende des jeweiligen Isolatorelementes 32 bzw. 34 allseitig als Kappe und bieten somit einen Schutz für das distale Ende des Isolatorelements 32 bzw. 34 gegen Abnutzung. Die Spitzen 42 und 44 können auch noch stärker ausgeprägt sein und das distale Ende der Isolatorelemente 32 und 34 noch weiter überragen als in Fig. 2 dargestellt ist. Die Spitzen 42 und 44 können auch so ausgebildet sein, daß sie distal in zwei pinzettenähnlichen Branchen enden, die ein feines Präparieren ermöglichen. Im Falle einer derartigen Ausgestaltung besteht dann eine hohe Hochfrequenzstrom-Leistungsdichte, so daß damit auch eine Schneidfunktion bzw. ein schneidenähnliches Verhalten des Instruments ermöglicht wird, ohne daß Scherenschneiden vorhanden wären.

Die Leiterelemente 36 und 38 weisen auf ihren einander gegenüber liegenden Oberflächen ferner eine Profilierung zum verbesserten Fassen in Form von Erhabungen 46 bzw. 48 auf, wobei die Erhabungen 46 gegenüber den Erhabungen 48 axial versetzt angeordnet sind. Die Erhabungen 46 und 48 sind in dem gezeigten Ausführungsbeispiel flächig ausgebildet, es kann jedoch auch eine Faßprofilierung in Form einer gezahnten Ausgestaltung der Leiterelemente 36 bzw. 38 in Betracht gezogen werden. Die Erhabungen 46 bzw. 48 sind ebenfalls metallisch und können mit den Leiterelementen 36 bzw. 38 einstückig verbunden oder auf diese aufgelötet sein. Wenn die Leiterelemente 36 bzw. 38 mit einer Verzahnung ausgebildet sind, kann die Oberfläche 40 des Isolatorelements 34 und ebenso die entsprechende Oberfläche des Isolatorelements 32 eine gezahnte Formgebung aufweisen, um die Leiterelemente 36 bzw. 38 formschlüssig mit den Isolatorelementen 32 bzw. 34 verbinden zu können.

Mit der Spitze 42 des Leiterelements 36 bzw. mit der Spitze 44 des Leiterelements 38 ist jeweils ein Fortsatz in Form eines Röhrchens 48 bzw. 50 leitend verbunden, die jeweils in das zugehörige Isolatorelement 32 bzw. 34 (vergleiche Fig. 3) in eine darin vorgesehene Bohrung eingesetzt sind.

Um die als Elektroden dienenden Leiterelemente 36 bzw. 38 mit Hochfrequenzstrom beaufschlagen zu können, sind zwei voneinander isolierte Stromzuleitungen 52 bzw. 54 vorgesehen, die sich durch den Rohrschaft 12 erstrecken, wobei ein distales Ende der Stromzuleitung 52 mit dem Leiterelement 36 und ein distales Ende der Stromzuleitung 50 mit dem Leiterelement 38 elektrisch leitend verbunden ist.

Die Stromzuleitungen 52 bzw. 54 sind in Form von dünnen, flexiblen und mit einem isolierenden Mantel umgebenen Drähten ausgebildet.

Das distale Ende 56 der Stromzuleitung 52 sowie das distale Ende 58 der Stromzuleitung 54 ist in dem jeweiligen Isolatorelement 32 bzw. 34 allseitig umschlossen eingebettet, wozu in die Isolatorelemente 32 bzw. 34 eine entsprechende durchgehende axiale Bohrung eingebracht ist. Dabei ist in das proximale Ende des Isolatorelements 32 bzw. in das proximale Ende des Isolatorelements 34 noch ein Teil 60 bzw. 62 des isolierenden Mantels der Stromzuleitungen 52 bzw. 54 eingeführt. Die distalen Enden 56 bzw. 58 sind bis zum distalen Ende der Isolatorelemente 32 bzw. 34 durchgeführt und dort in die Röhrchen 48 bzw. 50, die mit den Leiterelementen 36 bzw. 38 elektrisch leitend in Verbindung stehen, eingeführt.

Wie aus Fig. 3 ferner hervorgeht, überragen die Isolatorelemente 32 bzw. 34 den jeweiligen zugehörigen metallischen Grundkörper 28 bzw. 30 auch in Umfangsrichtung, so daß eine ausreichende Beabstandung der Leiterelemente 36 bzw. 38 von den metallischen Grundkörpern 28 bzw. 30 erreicht wird.

Im folgenden wird die gelenkige Verbindung der Maulteile 14 und 16 sowie der Betätigungsmechanismus zum Öffnen und Schließen der Maulteile 14 und 16 näher beschrieben.

Der metallische Grundkörper 28 des beweglichen Maulteils 14 weist an seinem proximalen Ende einen einstückig mit dem übrigen Teil des Grundkörpers 28 verbundenen, ebenfalls metallischen gegabelten Abschnitt 64 auf, von dem in Fig. 2 ein rechter Schenkel 66 zu sehen ist. Der Schenkel 66 ist mittels eines Zapfens 68, dessen innenliegendes Ende bündig mit dem Schenkel 66 abschließt, mit dem Rohrschaft 12 gelenkig verbunden. Ebenso ist der dem Schenkel 66 gegenüber liegende nicht dargestellte Schenkel mit einem entsprechenden Gelenkzapfen mit der gegenüber liegenden Seite des Rohrschafts 12 verbunden. Der Gelenkzapfen 68 sowie der gegenüber liegende nicht dargestellte Gelenkzapfen sind ebenfalls metallisch ausgeführt.

Die Stromzuleitungen 52 bzw. 54 sind durch den gegabelten Abschnitt 64 des metallischen Grundkörpers 28 des Maulteils 14 durchgeführt. Durch die gegabelte Ausführung des metallischen Grundkörpers 28 ist für die Durchführung der Stromzuleitungen 52 und 54 genügend Raum vorhanden.

In den gegabelten Abschnitt 64 greift ein Arm 70 ein, der mit dem Schenkel 66 und dem diesen gegenüber liegenden Schenkel über einen weiteren Gelenkzapfen 72 gelenkig verbunden ist. Der Arm 70 sowie der Gelenkzapfen 72 sind ebenfalls aus Metall gefertigt.

Der Arm 70 ist mit seinem proximalen Ende mit einem distalen Ende eines in dem Rohrschaft 12 angeordneten Zug- und Schubrohres 74 verbunden. Der Arm 70 weist dazu an seinem proximalen Ende eine Hülse 76 zur sicheren Verbindung mit dem Zug- und Schubrohr 74 auf. Das Zug- und Schubrohr 74 ist in dem Rohrschaft 12 axial verschieblich aufgenommen und reicht bis zum proximalen Ende des Rohrschafts 12, wo es mit dem beweglichen Griffteil 20 der Handhabe 18 verbunden ist. Das Zug- und Schubrohr dient als Kraftübertragungselement von dem beweglichen Griffteil 20 auf das Maulteil 14, um dieses zu öffnen oder zu schließen. Das bewegliche Griffelement 20 weist dazu einen Schenkelabschnitt 78 auf, der gegabelt ausgeführt ist, und in dessen Gabel ein Zapfen 80 eingreift, der wiederum mit dem Zug- und Schubrohr 74 mechanisch in Verbindung steht.

Durch Zusammendrücken der Griffelemente 20 und 22 wird das Zug- und Schubrohr 74 nach distal verschoben, wodurch das erste Maulteil 14 um den Gelenkzapfen 68 aus der dargestellten Offenlage zu dem zweiten Maulteil 16 geschwenkt wird, und umgekehrt. Das erste Griffelement 20 und das zweite Griffelement 22 sind über eine Blattfeder 82 und einen Schwenkarm 84 in ihre Spreizstellung, in der das Maulteil 14 seine Offenlage einnimmt, vorgespannt.

Am proximalen Ende des Rohrschafts 12 ist schließlich ein Stekkergehäuse 86 mit einem Kontaktfinger 88 angeordnet, zu dem die Stromzuleitungen 52 und 54 geführt sind. An das Steckergehäuse 86 kann ein nicht dargestellter Hochfrequenzstecker einer nicht dargestellten Hochfrequenzspannungsquelle angeschlossen werden, wobei dann die Stromzuleitung 52 mit dem einen Pol der Hochfrequenzspannungsquelle und die Stromzuleitung 54 mit dem anderen Pol der Hochfrequenzspannungsquelle verbunden wird, so daß an die Leiterelemente 36 und 38 eine Hochfrequenzspannung unterschiedlichen Potentials gelegt werden kann.

Aus der vorhergehenden Beschreibung ergibt sich, daß das gesamte Instrument 10 bis auf die Isolatorelemente 32 und 34 und das Steckergehäuse aus Metall gefertigt werden kann, wobei insbesondere die gelenkige Verbindung des ersten Maulteils 14 mit dem zweiten Maulteil 16, die durch den gegabelten Abschnitt 64 des metallischen Grundkörpers 28, den Gelenkzapfen 68, den Arm 70 und den Gelenkzapfen 72 gebildet wird, keine isolierenden Materialien aufweist, sondern vollständig aus metallischen Elementen besteht, so daß die gelenkige Verbindung eine hohe Stabilität besitzt.

## Patentansprüche

1. Bipolares medizinisches Instrument, mit einem Rohrschaft (12), mit zwei Maulteilen (14, 16) am distalen Ende des Rohrschafts (12), wobei die Maulteile (14, 16) gelenkig miteinander verbunden sind und jeweils eine mit Hochfrequenzstrom beaufschlagbare Elektrode ausbilden, wobei die Maulteile (14, 16) jeweils einen metallischen Grundkörper (28, 30) aufweisen, die miteinander gelenkig verbunden sind, wobei die metallischen Grundkörper (28, 30) auf ihrer einander zugewandten Seite jeweils ein Isolatorelement (32, 34) aufweisen, wobei mit den Isolatorelementen (32, 34) jeweils ein den zugehörigen metallischen Grundkörper (28, 30) nicht berührendes, die jeweilige Elektrode bildendes Leiterelement (36, 38) verbunden ist, wobei sich durch den Rohrschaft (12) zwei voneinander isolierte Stromzuleitungen (52, 54) bis zu den Leiterelementen (36, 38) erstrecken und mit diesen leitend verbunden sind, und wobei jedes Isolatorelement (32, 34) das damit verbundene Leiterelement (36, 38) von dem zugehörigen Grundkörper (28, 30) elektrisch isoliert.

2. Instrument nach Anspruch 1, **dadurch gekennzeichnet, daß** ein distales Ende (56, 58) der jeweiligen Stromzuleitung (52, 54)im zugehörigen Isolatorelement (32, 34) von diesem umschlossen angeordnet ist und mit einem in das Isolatorelement (32, 34) ragenden Fortsatz des Leiterelements (36, 38) leitend verbunden ist.

3. Instrument nach Anspruch 2, **dadurch gekennzeichnet, daß** das distale Ende (56, 58) der jeweiligen Stromzuleitung (52, 54) im Isolatorelement (32, 34) von dessen proximalen bis zu dessen distalen Ende durchgeführt und in ein in das distale Ende des Isolatorelements (32, 34) ragendes Röhrchen des Leiterelements (36, 38) eingeführt ist.

4. Instrument nach einem der Ansprüche 1 bis 3, **dadurch gekennzeichnet, daß** die Isolatorelemente (32, 34) den zugehörigen metallischen Grundkörper (28, 30) distal überragen und die Leiterelemente (36, 38) zumindest am distalen Ende der Isolatorelemente (32, 34) angeordnet sind und eine Spitze (42, 44) aufweisen.

5. Instrument nach Anspruch 4, **dadurch gekennzeichnet, daß** die Spitzen (42, 44) distal in pinzettenähnlichen Branchen enden.

6. Instrument nach Anspruch 4 oder 5, **dadurch gekennzeichnet, daß** die Leiterelemente (36, 38) das distale Ende der Isolatorelemente (32, 34) als Kappe umgeben.

7. Instrument nach einem der Ansprüche 1 bis 6, **dadurch gekennzeichnet, daß** die Leiterelemente (36, 38) als Plättchen ausgebildet sind und sich flächig im wesentlichen über die gesamte, dem jeweils gegenüberliegenden Maulteil (14, 16) zugewandte Oberfläche (40) der Isolatorelemente (32, 34) erstrecken.

8. Instrument nach einem der Ansprüche 1 bis 7, **dadurch gekennzeichnet, daß** die Isolatorelemente (32, 34) die metallischen Grundkörper (28, 30) in Umfangsrichtung überragen.

9. Instrument nach einem der Ansprüche 1 bis 8, **dadurch gekennzeichnet, daß** die Isolatorelemente (32, 34) von dem jeweiligen metallischen Grundkörper (28, 30) eingefaßt sind.

10. Instrument nach einem der Ansprüche 1 bis 9, **dadurch gekennzeichnet, daß** die Isolatorelemente (32, 34) mit dem metallischen Grundkörper (28, 30) verklebt sind.

11. Instrument nach Anspruch 10, **dadurch gekennzeichnet, daß** der Klebstoff hitze- und/oder feuchtigkeitsbeständig ist.

12. Instrument nach einem der Ansprüche 1 bis 11, **dadurch gekennzeichnet, daß** die Isolatorelemente (32, 34)aus einem Keramikwerkstoff, vorzugsweise einem Keramikwerkstoff hoher Härte und geringer Sprödigkeit gefertigt sind.

## Claims

1. A bipolar medical instrument, comprising a tubular shaft 12, two jaw parts (14, 16), disposed at the distal end of the tubular shaft (12), wherein the jaw parts (14, 16) are pivotally connected to one another and each form an electrode suppliable with high-frequency current, wherein the jaw parts (14, 16) each have a metallic base (28, 30) which are pivotally connected to one another, wherein the metallic bases (28, 30) each have an insulator element on the side facing one another, wherein respective conductive elements (36, 38) forming the respective electrode, which are not in contact with the associated metallic base (28, 30) are connected to the respective insulator elements (32, 34), and wherein two electrical lines (52, 54) insulated from one another extend through the tubular shaft (12) to the conductive elements (36, 38) and are electrically connected thereto, and wherein each insulator element (32, 34) isolates the respective conductive element (36, 38) connected thereto from the associated base (28, 30).

2. The instrument of claim 1, **characterized in that** a distal end (56, 58) of the respective electrical line (52, 54) is arranged in and enclosed by the associated insulator element (32, 34) and is conductively connected to a projection of the conductive element (36, 38) protruding into the insulator element (32, 34).

3. The instrument of claim 2, **characterized in that** the distal end (56, 58) of the respective electrical line (52, 54) is passed through the insulator element (32, 34) from the proximal to the distal end thereof and is inserted into a small tube of the conductive element (36, 38) protruding into the distal end of the insulator element (32, 34).

4. The instrument of anyone of claims 1 through 3, **characterized in that** the insulator elements (32, 34) extend distally beyond the associated metallic base (28, 30), and the conductive elements (36, 38) are arranged at least at the distal end of the insulator elements (32, 34) and have a tip (42, 44).

5. The instrument of claim 4, **characterized in that** the tips (42, 44) terminate distally in tweezer-like branches.

6. The instrument of claim 4 or 5, **characterized in that** the conductive elements (36, 38) surround the distal end of the insulator elements (32, 34) in form of a cap.

7. The instrument of anyone of claims 1 through 6, **characterized in that** the conductive elements (36, 38) are configured as small plates and extend substantially over the entire surface (40) of the insulator elements (32, 34), which surface (40) faces the opposite jaw part (14, 16), respectively.

8. The instrument of anyone of claims 1 through 7, **characterized in that** the insulator elements (32, 34) extend beyond the metallic bases (28, 30) in circumferential direction.

9. The instrument of anyone of claims 1 through 8, **characterized in that** the insulator elements (32, 34) are seated in the respective metallic base (28, 30).

10. The instrument of anyone of claims 1 through 9, **characterized in that** the insulator elements (32, 34) are glued with the metallic base (28, 30).

11. The instrument of claim 10, **characterized in that** the adhesive is heat and/or moisture resistant.

12. The instrument of anyone of claims 1 through 11, **characterized in that** the insulator elements (32, 34) are made of a ceramic material, preferably a ceramic material of high hardness and low brittleness.

## Revendications

1. Instrument bipolaire à usage médical comprenant une tige tubulaire (12) et deux parties de mâchoire (14, 16) à l'extrémité distale de la tige tubulaire (12), ces parties de mâchoire (14, 16) étant reliées de manière articulée et formant, dans chaque cas, une électrode soumise à un courant haute fréquence, les parties de mâchoire (14, 16) présentant chacune un corps de base (28, 30) métallique, les corps étant reliés de manière articulée, les corps de base (28, 30) métalliques présentant, sur leurs côtés qui se font mutuellement face, un élément isolant (32, 34), un élément conducteur (36, 38) formant chacune des électrodes, ne touchant pas le corps de base (28, 30) métallique auquel il est associé, étant relié dans chaque cas aux éléments isolants (32, 34), deux lignes d'amenée de courant (52, 54) isolées s'étendant à travers la tige tubulaire (12), jusqu'aux éléments conducteurs (36, 38) auxquels elles sont connectées de manière conductrice, et chaque élément isolant (32, 34) isolant électriquement l'élément conducteur (36, 38) auquel il est connecté, vis-à-vis du corps de base (28, 30) correspondant.

2. Instrument selon la revendication 1, **caractérisé en ce qu'**une extrémité distale (56, 58) de la ligne d'amenée de courant (52, 54) est montée dans l'élément isolant (32, 34) associé en étant entourée par lui, et est connectée de manière conductrice avec un prolongement (36, 38) de l'élément conducteur dépassant dans l'élément isolant (32, 34).

3. Instrument selon la revendication 2, **caractérisé en ce que** l'extrémité distale (56, 58) de la ligne d'amenée de courant (52, 54) montée dans l'élément isolant (32, 34) passe à travers lui, de son extrémité proximale à son extrémité distale, et est introduite dans un petit tube de l'élément conducteur (36, 38) qui dépasse dans l'extrémité distale de l'élément isolant (32, 34).

4. Instrument selon l'une des revendications 1 à 3, **caractérisé en ce que** les éléments isolants (32, 34) dépassent distalement du corps de base (28, 30) métallique qui leur est associé, et **en ce que** les éléments conducteurs (36, 38) sont montés au moins à l'extrémité distale des éléments isolants (32, 34) et présentent une pointe (42, 44).

5. Instrument selon la revendication 4, **caractérisé en ce que** les pointes (42, 44) se terminent distalement par des branches semblables à des pincettes.

6. Instrument selon la revendication 4 ou 5, **caractérisé en ce que** les éléments conducteurs (36, 38) entourent l'extrémité distale des éléments isolants (32, 34) en formant un capuchon.

7. Instrument selon l'une des revendications 1 à 6, **caractérisé en ce que** les éléments conducteurs (36, 38) sont conformés en plaquettes et s'étendent de façon plane, sensiblement sur toute la surface (40) des éléments isolants (32, 34) qui est orientée vers la partie de mâchoire (14, 16) située en regard.

8. instrument selon l'une des revendications 1 à 7, **caractérisé en ce que** les éléments isolants (32, 34) dépassent, dans la direction de la circonférence, des corps de base (28, 30) métalliques.

9. Instrument selon l'une des revendications 1 à 8, **caractérisé en ce que** les éléments isolants (32, 34) sont entourés par le corps de base (28, 30) métallique qui leur est associé.

10. Instrument selon l'une des revendications 1 à 9, **caractérisé en ce que** les éléments isolants (32, 34) sont collés avec le corps de base (28, 30) métallique.

11. Instrument selon la revendication 10, **caractérisé en ce que** la colle résiste à la chaleur et/ou à l'humidité.

12. Instrument selon l'une des revendications 1 à 11, **caractérisé en ce que** les éléments isolants (32, 34) sont constitués en matière céramique, de préférence en matière céramique de grande dureté et de faible fragilité.
